# EUROPEAN PATENT SPECIFICATION

(11) **EP 4 338 775 B1**
(45) Date of publication and mention of the grant of the patent: **01.04.2026**
(21) Application number: 22196394.5
(22) Date of filing: 19.09.2022
(51) Int. Cl.: A61M 16/12

(54) **MANUAL ARTIFICIAL RESPIRATION BAG COMPRISING A VENTURI DEVICE**
MANUELLER KÜNSTLICHER BEATMUNGSBEUTEL MIT VENTURI-VORRICHTUNG
SAC DE RESPIRATION ARTIFICIELLE MANUEL COMPRENANT UN DISPOSITIF VENTURI

(43) Date of publication of application: 20.03.2024
(73) Proprietor: Air Liquide Medical Systems, 92182 Antony Cedex (FR); Air Liquide Medical Systems S.r.l., 20152 Milano (IT)
(72) Inventor: DE BEAUFORT, Eloise, 92182 Antony Cedex (FR); LESIMPLE, Arnaud, 92182 Antony Cedex (FR); BROC, Alexandre, 92182 Antony Cedex (FR); ZADRA, Davide, 25073 Bovezzo (IT); ALBERICI, Luca, 25073 Bovezzo (IT); RICHARD, Jean-Christophe, 92182 Antony Cedex (FR)
(74) Representative: Air Liquide

(56) References cited:
- BE-B1- 1 023 576
- GB-A- 2 407 043
- US-A- 3 262 446
- US-A- 3 913 607
- US-A- 4 886 055
- US-A1- 2018 333 555
- US-A1- 2021 205 566

## Description

The present invention relates to a manual artificial respiration bag, such as manual resuscitation devices/systems, resuscitators or the like, comprising a venturi device that can be used for providing a respiratory gas, namely oxygen or an air/oxygen mixture, to a person, i.e. a patient, especially a person in state of cardiac arrest or any other patient in need of being artificially ventilated by such a manual artificial respiration bag.

A manual resuscitation bag can be used for providing a respiratory gas, such as air, oxygen or a mixture thereof, to a person in need of a respiratory assistance, typically a patient.

Classical manual artificial respiration bags are used for providing a respiratory gas, such as air or an air/O₂ mixture, to a patient, during his/her transportation or transfer from a first place, such as an operation room or a radiotherapy room, to a second place, such as a recovery room or a bedroom, or vice versa. Examples of such bags are given by US-A-3,063,620, US-A-2017/0157348, US-A-4,501,271 or US-A-2,834,339. However, such classical manual artificial respiration bags are not suitable for providing a respiratory gas to a person in state of cardiac arrest as they are not compatible or well-adapted with the cardiac massage that is done on the person.

Some manual artificial respiration bags, also called manual resuscitation bags, have been specifically designed for artificially ventilating a person in state of cardiac arrest, with air or an air/O₂ mixture, while thoracic compressions (TC), i.e. successive compressions and decompressions, are exerted by a rescuer on the thoracic cage of said person for restoring gas exchanges in the lungs and a blood circulation in the body and toward the organs, especially to the brain of the patient. Examples of such manual resuscitation bags are given by WO2019/001751, WO2019/001752, WO2017/096286, WO2015/041396, WO2005/035065 and EP-A-0743075.

Further, EP-A-3884982 and EP-A-3884983 disclose improved multifunctional manual artificial respiration bags that can be used in various situations, such as transportation/transfer of patients, persons in state of cardiac arrest that undergo thoracic compressions... Such an improved manual artificial respiration bag comprises an air entry and an oxygen entry for providing both air and oxygen to the bag, thereby allowing delivering either air, oxygen or mixtures thereof to the patient in need thereof.

BE1023576B1 discloses a manual resuscitation bag with an ambient air-oxygen blending device having a Venturi nozzle.

US2018/333555A1 discloses modular adjustable ambient air-oxygen blending devices with Venturi nozzles optionally comprising threads.

Further adjustable ambient air-oxygen blending devices with Venturi nozzles are known for example from US4886055A, GB2407043A, US2021/205566A1 and US3913607A.

When ventilating a person in state of cardiac arrest with such a manual respiration bag, it is necessary to provide a fraction of inspired oxygen (FiO₂) as high as possible, typically of about 100% of oxygen, for maximizing the oxygenation of his/her blood, while the cardiac pump is stopped.

However, after a return to a spontaneous circulation (ROSC), i.e. of a blood circulation perfusing the body resulting from the resumption of a cardiac rhythm, it is advocated to lower the FiO₂, i.e. the quantity of oxygen that is provided by the manual respiration bag, for taking into account the variation of the oxygen saturation of the patient, namely the concentration of oxygen into the blood of the patient.

This is currently done in reducing the flowrate of oxygen that is provided to the manual respiration bag, i.e. lowering the quantity of oxygen entering into the manual respiration bag and provided afterwards to the patient.

Unfortunately, reducing the flowrate of oxygen provided to the manual respiration bag is not ideal and/or satisfying. Indeed, operating an efficient monitoring the FiO₂ level based only on the oxygen flowrate is quite impossible and not reliable as if the FiO₂ level depends on the oxygen flowrate, it also depends on additional parameters, such as the insufflated gas volumes, the frequency of insufflation, the characteristics of the manual respiration bag used... As a consequence, none of the existing manual respiration bags is able to provide accurate and precise levels of FiO₂.

In other words, with known manual respiration bags, the amount of oxygen provided to the patients is random, especially for amounts less than 100 vol.%.

In this context, a goal of the present invention is to provide an improved manual artificial respiration bag allowing a more accurate control of the proportion of oxygen provided to the patients, especially when the proportion of oxygen has to be less than 100 vol.%, typically less than 80 vol.%, for instance of about 50 vol.%, especially in case of ROSC while ventilating a person having had a cardiac arrest.

In other words, there is a need for a manual respiration bags that can provide at least two accurate and precise levels of FiO₂ for ventilating persons suffering from a cardiac arrest or from another respiratory pathology or medical condition requiring at least two different amounts of oxygen, i.e. various

A solution according to the present invention concerns a manual artificial respiration bag as defined in the appended independent claim 1. Preferred embodiments are matter of the dependent claims.

A manual artificial respiration bag according to the present disclosure comprises:
- a deformable bag, i.e. flexible bag, comprising a gas inlet, a gas outlet and an inner volume for a respiratory gas, namely oxygen or an air/oxygen mixture,
- an upstream conduct element fluidly connected to the gas inlet of the deformable bag, and comprising a gas entry port, and
- a downstream conduct element fluidly connected to the gas outlet of the deformable bag, comprising an exhaust valve comprising an exhaust port, characterized in that it further comprises a venturi device comprising :
- a main body comprising a venturi nozzle,
- at least one oxygen inlet for providing oxygen,
- at least one air inlet for providing air,
- a mixing chamber for mixing therein oxygen and air provided by said at least one oxygen inlet and said at least one air inlet, thereby obtaining an air/oxygen mixture, and
- gas selection means for selecting oxygen provided by the at least one oxygen inlet or an air/oxygen mixture provided by the mixing chamber as a desired gas to be provided to the venturi nozzle, and
wherein the venturi nozzle is in fluid communication with the upstream conduct element, via the gas entry port arranged in said upstream conduct element, for providing the desired gas to the upstream conduct element.

Depending on the embodiment, the venturi device of the manual respiration bag according to the present disclosure can comprise of one or several of the following features:
- the gas selection means of the venturi device comprise a rear member comprising said at least one oxygen inlet, said at least one air inlet, and said mixing chamber.
- the venturi nozzle firmly fixed to the upstream conduct element, i.e. integral with the upstream conduct element.
- the gas entry port is the unique port of the upstream conduct element used for providing oxygen or an air/oxygen mixture, i.e. the desired gas.
- the rear member is mobile with respect to the main body between at least :
   ∘ a closed position wherein the rear member provides oxygen as the desired gas to the venturi nozzle, and
   ∘ an open position wherein the rear member provides an air/oxygen mixture as the desired gas to the venturi nozzle.
- the rear member is mobile in rotation and/or in translation with respect to the main body of the venturi device for allowing a user to manually select the desired open or the closed position.
- the rear member is preferably mobile according to a helicoidal motion with respect to the main body of the venturi device.
- the main body of the venturi device comprises an inner compartment containing the venturi nozzle.
- the main body and the rear member are mobile with respect to the other for controlling, i.e. selecting, the type of gas, i.e. desired gas, that has to be provided by the venturi device to the deformable reservoir, namely either oxygen or an air/oxygen mixture.
- the main body and the rear member are made of polymer.
- the gas selection means of the venturi device comprise the rear member and at least a part of the main body cooperating together for selecting the type of gas, i.e. desired gas.

- the rear member is a gas-providing member as it provides gas(es), namely oxygen and/or air, including an air/oxygen mixture.
- the closed position and the open position are stable positions.
- the rear member (or the main body) can move back and forth with respect to the main body (or the rear member), while (hand-)actuated according to preferably a helicoidal motion by the user, i.e. while turned clockwise or counterclockwise by the user, such as a rescuer or any other medical staff member.
- the rear member cooperates with the venturi nozzle arranged in the main body for providing oxygen or an air-oxygen mixture to the venturi nozzle, in particular to the nozzle inlet of the venturi nozzle.
- the rear member is mobile with respect to the main body along a helicoidal path, i.e. according to a helicoidal motion, namely a combination of a rotation and a translation, when (hand)actuated by the user, especially when it is turned or rotated clockwise or counter-clockwise.
- the main body comprises at least one elongated groove and the rear member comprises at least one guiding member, or vice versa, preferably at least 2 elongated grooves cooperating with at least 2 guiding members.
- said at least one guiding member is inserted into said at least one elongated groove so as to cooperate with said at least one elongated groove for guiding the motion, in particular a helicoidal motion, of the rear member with respect to the main body, while the rear member is actuated, i.e. at least rotated / turned around axis (XX), by a user with respect to the main body.
- preferably, the main body comprises at least two elongated grooves and the rear member comprises at least two guiding members, or vice versa, in particular two elongated grooves and two guiding members, each guiding member cooperating with one elongated groove.
- the guiding members and the elongated grooves are configured so that each guiding member travels into one corresponding elongated groove, when the rear member is actuated (i.e. turned), clockwise or counterclockwise, with respect to the main body, or vice versa, by a user.
- the two elongated grooves are diametrically-oppositely arranged on the main body.
- the two guiding members are diametrically-oppositely arranged on the rear member.
- each elongated groove constitutes a guide or path that guides the guiding member, while the venturi device is actuated by the user.
- each elongated groove is arranged to form a slot traversing the peripheral wall of the main body, i.e. a traversing slot or the like, preferably traversing the peripheral wall of the inlet chamber of the main body.
- each guiding member comprises or is a protrusion arranged on the peripheral outer surface of a front portion of the rear member.
- each protrusion forms a dent, a pin, a finger or any other similar small structure projecting away from the peripheral outer surface of the front portion of the rear member.
- the venturi nozzle comprises a nozzle inlet and a nozzle outlet.
- the venturi nozzle further comprises an inner nozzle channel configured for conveying the gas, i.e. oxygen or air/oxygen, from the nozzle inlet to the nozzle outlet.
- the inner nozzle channel axially-traverses the venturi nozzle.
- the inner compartment is divided by an inner wall in 2 sub-compartments, namely an inlet chamber, also called upstream chamber, and an outlet chamber, also called downstream chamber.
- the main body has an axially-elongated shape, i.e. along axis (XX).
- the venturi nozzle is arranged through, i.e. traverses, said inner wall.
- the inner wall arranged in the inner compartment of the main body has a ring shape or the like.
- the inner wall is radially-arranged in the inner compartment of the main body, i.e. the inner wall projects radially in the inner compartment.
- the outlet chamber comprises the gas outlet of the venturi nozzle.
- the outlet chamber is open to, i.e. it fluidly communicates with, the exterior of the main body, especially with the entry port.
- the inlet chamber is open to, i.e. it fluidly communicates with, the mixing chamber of the rear member.
- the inlet chamber of the inner compartment of the main body is in fluid communication with the mixing chamber of the rear member.
- the oxygen-containing gas (i.e. O₂ or air/O₂) can only travel through the venturi nozzle for reaching the gas outlet. In other words, there is no direct fluid communication between the inlet chamber and the outlet chamber, except via the venturi nozzle.
- the main body has a generally-tubular shape, preferably a cylindrical shape.
- the main body has an outer diameter of about between 25 and 40 mm, and a length of about between 60 and 110 mm.
- a front portion of the rear member is inserted into at least a part of the inlet chamber of the main body.
- the rear member is at least partially inserted into the main body so as to be mobile according to a helicoidal motion therein, while (hand)actuated by the user.
- the front portion of the rear member is cylindrical, i.e. tubular, and at least a part of the inlet chamber of the main body is cylindrical, so that said cylindrical front portion of the rear member is mobile according to a helicoidal motion into said cylindrical part of the inlet chamber of the main body.
- the rear member comprises a tubular portion closed at one end by a blind bottom, i.e. a tubular portion terminated by a closed end.
- the rear member comprises a cup-like shape portion comprising the tubular portion terminated by the closed end.
- the rear member comprises a cylindrical body terminated by an open end at a first end and a closed end, i.e. a blind bottom, at a second end.
- the rear member has a generally-tubular shape having an outer diameter of about between 20 and 40 mm, and a length of about between 40 and 70 mm.
- the rear member comprises, i.e. is traversed by, an oxygen feeding conduct comprising an outer conduct portion (i.e. upstream portion) and an inner conduct portion (i.e. downstream portion), for providing oxygen.
- the oxygen feeding conduct is axially-arranged in the rear member.
- the closed end, i.e. the blind bottom or closing wall, of the rear member is traversed by the oxygen feeding conduct.
- the outer conduct portion of the oxygen feeding conduct comprises the oxygen inlet.
- the inner conduct portion of the oxygen feeding conduct comprises an oxygen delivery port.
- the oxygen feeding conduct is axially-traversed by a lumen for conveying oxygen from the oxygen inlet to the oxygen delivery port thereby providing oxygen coming from an external oxygen source.
- the outer conduct portion of the oxygen feeding conduct comprises connector means, such as a specific outer shape, for fluidly connecting an oxygen line thereto, preferably an oxygen line fluidly connected to an external oxygen source, such as an oxygen gas cylinder or an oxygen wall-plug fed by a gas network.
- the inner conduct portion of the oxygen feeding conduct is arranged so as to (axially-) project into the mixing chamber of the rear member.
- the oxygen delivery port is arranged at a proximal free end of the inner conduct portion of the oxygen feeding conduct.
- the outer conduct portion of the oxygen feeding conduct is arranged so as to (axially-) project outwardly of the rear member, i.e. outside of it.
- the oxygen inlet is arranged at a distal free end of the outer conduct portion of the oxygen feeding conduct.
- the oxygen feeding conduct is in fluid communication, in particular the lumen of the inner and outer conduct portions, with the mixing chamber, when the rear member is in the open position.
- the oxygen feeding conduct is in fluid communication, in particular the lumen of the inner and outer conduct portions, with the inner nozzle channel of the venturi nozzle, when the rear member is in the closed position.
- the mixing chamber of the rear member has an inner cylindrical shape.
- the outer diameter of the cylindrical front portion of the rear member is approximately equal to the inner diameter of the cylindrical part of the inlet chamber of the main body, i.e. the outer diameter of the cylindrical front portion is slightly less than the inner diameter of the cylindrical part of the inlet chamber so that the rear member is mobile, preferably according to a helicoidal motion, into said main body.
- the rear member comprises several air inlets or air ports for providing air.
- the rear member comprises a unique oxygen inlet or ports for providing gaseous oxygen.
- several air inlets are arranged around the oxygen feeding conduct, in particular in the blind bottom.
- the inlet chamber of the main body is delimited by a peripheral wall.
- the inlet chamber of the main body is in fluid communication with the mixing chamber of the rear element.
- the venturi nozzle comprises a first portion comprising the nozzle inlet.
- the first portion is a cylindrical or a convergent portion.
- the venturi nozzle further comprises a second portion comprising the nozzle outlet.
- the second portion is a divergent portion, i.e. it has a divergent inner shape (i.e. an increasing inner diameter).
- the first portion is fluidly connected to the second portion of the venturi nozzle.
- the divergent portion is arranged between the first portion of the nozzle channel (i.e. cylindrical portion) and the nozzle outlet, i.e. a gas exit of the venturi nozzle.
- the first portion and the second portion of the venturi nozzle form the inner nozzle channel for conveying the gas, i.e. oxygen or air/oxygen.
- the nozzle inlet is located at the entry of the inner nozzle channel and the nozzle outlet is located at the exit of the inner nozzle channel so that the nozzle inlet is fluidly connected to the nozzle outlet via the inner nozzle channel.
- the gas circulates into the venturi nozzle from the first portion, i.e. a gas entry portion, having preferably a circular or convergent shape, to the second portion, i.e. a gas exit portion, having preferably a divergent shape.
- in the closed position, the proximal free end of the inner conduct portion of the oxygen feeding conduct of the rear member is inserted into the first portion of the inner nozzle channel of the venturi nozzle so as to deliver oxygen directly into the venturi nozzle.
- in the open position, the proximal free end of the inner conduct portion of the oxygen feeding conduct of the rear member is not inserted into the first portion of the inner nozzle channel of the venturi nozzle, but delivers a flow of oxygen into the mixing chamber so as to generate an air/oxygen mixture into the mixing chamber of the venturi nozzle.
- in the open position, the proximal free end of the inner conduct portion of the oxygen feeding conduct of the rear member is positioned into the mixing chamber, i.e leads to or ends into it, for providing a flow of oxygen to the mixing chamber so as to generate an air/oxygen mixture into said mixing chamber.
- in the open position, air provided by the air inlet(s) is sucked into the mixing chamber of the rear member by the flow of oxygen provided by the oxygen feeding conduct of the rear member.
- each elongated groove of the main body, preferably each elongated slot, comprises a curved linear portion terminated by two end lodgings.
- each end lodging comprises an abutment, preferably each abutment is located at a site of junction between the elongated groove and each end lodging.
- the end lodgings and the abutments cooperate with the protrusions for setting, i.e. positioning, the venturi device in the open or in the closed positions, namely for ensuring a stable positioning of the rear member with respect to the main body in one or the other of the two stable positions, i.e. the open or closed stable positions.
- the curved linear portion of each elongated groove, preferably of each elongated slot, is diagonally-arranged in the peripheral wall of the main body, namely in the peripheral wall of the inlet chamber of the main body.
- each elongated groove, preferably of each elongated slot, has a length of about between 20 mm and 30 mm.
- each elongated groove, preferably of each elongated slot, has a width of about between 3.5 mm and 5 mm.
- each guiding member, i.e. each a protrusion, has a height of about between 1.5 and 3 mm.
- the venturi device is designed for providing (at least) a first amount of about 100 vol% of oxygen, i.e. a pure oxygen flow or a quasi-pur oxygen, for instance an oxygen amount of at least 95 vol.%, i.e. of between 95 and 100 vol.%.
- the venturi device is further designed for providing (at least) a second amount of oxygen contained in the oxygen/air mixture obtained that is less than 100 vol.%, resulting from the dilution of oxygen with ambient air in the mixing chamber of said venturi device, preferably the second amount of oxygen is less than 70 vol.%, more preferably less than 60 vol.%, and of at least 40 vol.%.
- the venturi device is designed for providing a second amount of oxygen (i.e. in an oxygen/air mixture) that is less the first amount of oxygen (i.e. an oxygen flow).
- typically the second amount of oxygen contained in the oxygen/air mixture is of between 45 and 55%, preferably of about 50 vol%.

Further, depending on the embodiment, a manual respiration bag according to the present disclosure can comprise of one or several of the following additional features:
- the manual artificial respiration bag further comprises an upstream one-way valve arranged into the upstream conduct element.
- the upstream one-way valve is configured for allowing a flow of gas to pass through said upstream one-way valve only toward the deformable bag.
- the upstream one-way valve is arranged between the deformable bag and the PEP exhaust valve,.
- the manual artificial respiration bag further comprises a downstream one-way valve arranged into the downstream conduct element, said downstream one-way valve being configured for allowing a flow of gas to pass through said downstream one-way valve only toward the patient.
- the downstream conduct element of the manual artificial respiration bag further comprises further comprises an exhaust valve with an exhaust port for venting to the atmosphere, gas coming out of the lungs of the patient, i.e. CO₂-enriched gas.
- the exhaust valve communicates with the lumen, i.e. inner volume, of the downstream conduct element.
- a mobile port-closing means (i.e. a mobile port-closing device), actuatable by the user, cooperates with the exhaust port of the exhaust valve for at least partially closing said exhaust port thereby controlling the flow of gas passing through the exhaust port of the exhaust valve.
- the mobile port-closing means are arranged on a mobile support-structure actuatable by the user.
- the mobile port-closing means at least partially close said exhaust port in response to an (hand-) actuation of the mobile support-structure by the user, typically to a rotation or a translation of the support-structure by the user.
- the mobile port-closing means cooperate with the exhaust port of the exhaust valve for partially closing said exhaust port thereby limiting the flow of gas passing through said exhaust port of the exhaust valve, i.e. during the use of the manual artificial respiration bag.
- the exhaust port of the exhaust valve is more or less open depending on the position of the mobile closing flap or wall of the support-structure, i.e. the exhaust port is at least partially closed when the support-element is actuated by the user, e.g. rotated.
- the support-structure carrying the port-closing means is rotatable, pivotable or translatable.
- the support-structure carrying the port-closing means is rotatable, i.e. configured for being mobile in rotation, when turned clockwise or counterclockwise by a user.
- the mobile port-closing means comprise a closing flap or wall, or the like.
- the closing flap or wall is carried by and/or integral with the mobile support-structure.
- the support-structure and the closing flap or wall are molded in one-piece.
- the mobile support-structure is coupled to (e.g. arranged on) the downstream conduct element.
- according to other embodiment, the port-closing means are arranged on the exhaust valve.
- the downstream conduct element comprises an inner passage, i.e. inner volume or lumen, for conveying gas.
- the manual artificial respiration bag can further comprise an over-pressure valve arranged into the downstream conduct element, such as a pressure relief valve.
- the downstream one-way valve comprises a valve-support arranged into the downstream conduct element, i.e. in its lumen, and a flexible valve body, preferably the valve-support is rigid.
- the flexible valve body has an umbrella-shape or any other suitable form, and/or is made of a flexible material, such as an elastomer.
- the flexible valve body has an umbrella-shape comprising a disk-shape body and a rod element integral with said disk-shape body, preferably fixed at the center of said disk-shape body and projecting away from said disk-shape body.
- the valve-support comprises a support orifice.
- the rod element of the flexible valve body traverses the support orifice of the valve-support, i.e. the rod element is lodged into said support orifice
- the upstream conduct element of the manual bag comprises a PEP exhaust valve fluidly communicating with the ambient atmosphere for venting gas to the atmosphere, when the gas pressure, into the upstream conduct element, exceeds a given pressure threshold.
- the upstream conduct element further comprises an safety admission valve in fluid communication with the ambient atmosphere for allowing ambient air to enter into the upstream conduct element for safety reasons, i.e. in case where the quantity of gas available into the upstream conduct element is insufficient for ventilating the patient, for instance in case of failure of the venturi device or the like.
- the PEP exhaust valve arranged in the upstream conduct element comprises PEP-setting means for setting a desired pressure threshold.
- the upstream conduct element further comprises a reservoir port for fluidly connecting a flexible gas reservoir thereto, such as a plastic pocket or the like.
- it further comprises a flexible gas reservoir fluidly connected to the reservoir port of the upstream conduct element.
- the downstream conduct element further comprises an interface port for fluidly connecting a respiratory interface thereto.
- the upstream conduct element and the downstream conduct element of the manual bag are made of polymer material, i.e. plastic or the like.
- a respiratory interface is fluidly connected to the interface port of the downstream conduct element, preferably by means of a ball-head connector or the like.
- it can further comprise a flow-restriction element arranged into the downstream conduct element.
- the PEP exhaust valve comprises a valve body and means for setting a desired pressure threshold comprising a rotatable member, actuatable by the user, arranged on the valve body and cooperating with pressure adjusting means arranged into the valve body.
- the opening pressure of the PEP exhaust valve is of between 0 cm H₂O and 30 cm H₂O, preferably of between 0 cm H₂O and 15 cm H₂O.
- the PEP exhaust valve comprises markings corresponding to several settable pressure values, in particular several settable pressure values of between 0 cm H₂O and 30 cm H₂O, for instance several pressure values comprising 0, 5 and 10 cmH₂O.
- the rotatable member of the PEP exhaust valve comprises an inner axially-projecting bulb cooperating with the pressure adjusting means arranged into the valve body.
- the pressure adjusting means arranged into the valve body comprise a piston head, a spring element and a valve seat cooperating with the piston head for adjusting the pressure threshold.
- the upstream conduct element comprises a inner passage or lumen.
- the valve body of the PEP exhaust valve is in fluid communication with the lumen of the first upstream conduct element.
- it further comprises a gas delivery conduct comprising the interface port, in fluid communication with the downstream conduct element for conveying at least part of the gas circulating into the gas conduct to a patient interface.
- the patient interface comprises of a respiratory mask or a tracheal cannula or probe.
- the overpressure valve, such as a pressure relief valve, arranged in the downstream conduct element is configured to vent to the atmosphere at least part of the gas present in the gas conduct, when the gas pressure in the downstream conduct element exceeds a given value, for instance of about 40 cmH₂O or any suitable pressure.

Some embodiments according to the present invention are shown in the enclosed Figures, among which:
- Figure 1 represents a side view of an embodiment of a manual artificial respiration bag according to the present invention, and
- Figures 2 to 8 are different views, including sectional lateral and views, of an embodiment of a venturi device equipping a manual artificial respiration bag according to the invention.

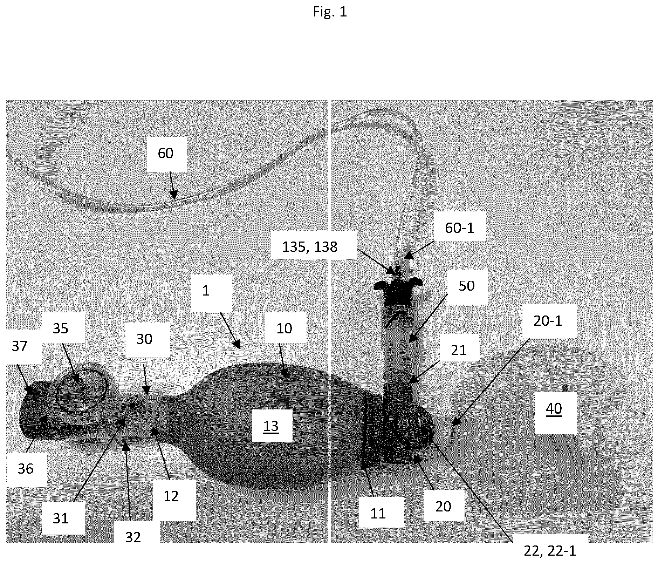

Figure 1 represents a side view of an embodiment of a manual artificial respiration bag 1 according to the present invention.

Said manual artificial respiration bag 1 comprises a deformable bag 10, i.e. flexible hollow bag or reservoir, comprising an inner volume 13 for receiving a respiratory gas, i.e. oxygen or a mixture of air and oxygen, a gas inlet 11 for allowing the gas to enter into said inner volume 13, and a gas outlet 12 for allowing the gas to exit said inner volume 13 of the deformable bag 10.

In use, a user, typically a rescuer, e.g. a physician or similar, can exert a manual-pressure on the deformable bag 10, i.e. manually squeeze it, for deforming it and thus expelling the gas contained therein, via the gas outlet 12, toward a patient in need thereof, such as a person in a state of cardiac arrest that should be artificially ventilated.

The deformable bag 10 is typically made of flexible material, typically a polymer material and has an inner volume 13 of preferably less than 2 L (i.e. when filled with water).

As shown in Figure 1, the manual artificial respiration bag 1 further comprises an upstream conduct element 20 fluidly connected to the gas inlet 11 of the deformable bag 10, and a downstream conduct element 30 fluidly connected to the gas outlet 12 of the deformable bag 10. Both upstream and downstream conduct elements 20, 30 comprise a generally-tubular shape comprising a lumen, i.e. inner passage, for receiving and conveying the gas flow.

Preferably, the upstream conduct element 20 comprises a PEP exhaust valve 22 fluidly communicating with the ambient atmosphere for venting gas (i.e. any over-pressure) to the atmosphere when the gas pressure, into the upstream conduct element 20, i.e. in its lumen, exceeds a given pressure threshold. In other words, the PEP exhaust valve 22 prevents gas overpressures in the deformable bag 10 and/or in the upstream conduct element 20 fluidly connected to the gas inlet 4 of the deformable bag 2.

The PEP exhaust valve 22 comprises a rotatable member 22-1, such as a rotating knob or the like, actuatable by a user, namely a rescuer, a valve body and means for setting a desired pressure threshold including pressure adjusting means arranged into the valve body. Said pressure adjusting means can comprise a piston head, a spring element, such as a cylindrical spring, and a valve seat cooperating with the piston head for adjusting the pressure threshold. The PEP exhaust valve 22 further comprises several markings corresponding to several settable pressure values, typically overpressure values of between 0 and 30 cm H₂O. In this aim, the rotatable member 22-1 further comprises an inner axially-projecting bulb, which cooperates with the pressure adjusting means for adjusting the pressure threshold. Such a PEP exhaust valve 22 has a known architecture.

In known manual artificial respiration bags, as taught by EP-A-3884983, the upstream conduct element generally comprises an air admission valve in fluid communication with the ambient atmosphere, for providing ambient air to the upstream conduct element, and an oxygen port for connecting an oxygen source thereto, such as an oxygen cylinder, for providing additional oxygen to the upstream conduct element and forming an oxygen/air mixture therein.

Instead, in the manual artificial respiration bag of Figure 1 according to the present invention, the upstream conduct element 20 comprises a unique gas entry port 21, i.e. a common and unique inlet, that is used for providing the desired gases to the lumen of the upstream conduct element 20, namely either pure oxygen or an oxygen/air mixture.

More precisely, both desired gases, i.e. pure oxygen or an oxygen/air mixture, are provided by a venturi device 50 that is arranged in the gas entry port 21 of the manual artificial respiration bag according to the present invention, as shown in Figure 1. In other words, a venturi device 50 is arranged so as to be in fluid communication with the unique gas entry port 21 for delivering either pure oxygen or an air/oxygen mixture to the upstream conduct element 20 of the manual artificial respiration bag according to the present invention. An embodiment of the venturi device 50 equipping the manual artificial respiration bag 1 according to the invention is detailed below.

Further, an oxygen source, such as an oxygen gas cylinder (not shown) or any other O₂ source, is fluidly connected to the venturi device 50 by means of a gas line 60 comprising a gas connector 60-1 or plug, such as a flexible hose or the like, as shown in Figure 1. The gas connector 60-1 is arranged at a free end of the gas line 60 and fluidly connected to connector means 138 arranged on the oxygen feeding conduct 135 of the venturi device 50 for providing oxygen gas to the venturi device 50, as explained below.

The manual artificial respiration bag according to the present invention of Figure 1 further comprises an upstream one-way valve (not visible) that is arranged into the upstream conduct element 20 between the deformable bag 10 and the PEP exhaust valve 22 and/or the unique gas entry port 21 in fluid communication with the venturi device 50.

The upstream one-way valve is configured for allowing the flow of respiratory gas provided by the unique gas entry port 21 and the venturi device 50 to pass through said upstream one-way valve only toward the deformable bag 10, i.e. to circulate in the direction of the deformable bag 10.

Similarly, a downstream one-way valve (not shown) is arranged into the downstream conduct element 30, i.e. into its lumen, for instance between the over-pressure valve 31 (when present), such as a pressure relief valve, and an exhaust valve 35, and is configured for allowing a flow of respiratory gas provided by the deformable bag 10 to pass through said downstream one-way valve only toward the patient. In other words, the role of the downstream one-way valve is to control the way that the gas circulates into the downstream one-way valve 30.

Said downstream one-way valve can comprise for instance a valve support arranged into the downstream conduct element 30 and a flexible valve body, which can have an umbrella-shape comprising a disk-shape body and a rod element integral with said disk-shape body, whereas the valve-support comprises a support orifice, the rod element of the flexible valve body traversing said support orifice of the valve-support, i.e. the rod element is positioned into the support orifice so as to be guided.

As already mentioned, the gas exits the deformable bag 10, via the gas outlet 12, upon manual pressures/squeezing operated by a rescuer, such as a physician or any other medical staff member, on the flexible peripheral wall(s) of the deformable bag 10, and then travels in the downstream conduct element 30, i.e. into its lumen, toward the patient.

Once the rescuer releases the deformable bag 10, i.e. stops exerting a manual squeezing of the deformable bag 10, the bag 10 is immediately refilled by "fresh" gas, i.e. oxygen or an air/oxygen mixture, provided by the venturi device 50 and/or a flexible gas reservoir 40 that replaces the quantity or volume of gas that has been provided to the downstream conduct element 30.

Actually, the upstream conduct element 20 further comprises a reservoir port 20-1 for fluidly connecting a flexible gas reservoir 40 thereto, as shown in Figure 1, such as a flexible polymer pocket or the like. Such a flexible gas reservoir 40 is used for storing a part of the gas provided by the unique gas entry port 21 and the venturi device 50, and providing gas when required, e.g. for refilling the the deformable bag 10. The flexible gas reservoir 40 constitutes a gas buffer or the like.

Furthermore, the downstream conduct elements 30 may also optionally comprise an over-pressure valve 31 (in some embodiments, such an over-pressure valve may be not necessary) for venting to the atmosphere any overpressure in said downstream conduct elements 30, i.e. in its lumen.

Preferably, the upstream conduct element 20 and/or the downstream conduct elements 30 are made of polymer material(s), such as plastic material(s) or the like.

In other words, the respiratory gas, such as air or an air/O₂ mixture, flowing out of the deformable bag 10, when squeezed by a medical staff member, passes through the downstream conduct element 30 that is fluidly connected to the gas outlet 12 of the deformable bag 10, and is subsequently delivered to the patient's airways, by means of a respiratory interface (not shown), such as a facial mask, a laryngeal mask, an endotracheal tube or the like that fluidly connected to an interface port 32 arranged in the downstream conduct element 30, as illustrated in Figure 1, for instance by means of a ball-head hollow connector or any other suitable tubular-connector.

The interface port 32 of the downstream conduct element 30 can be arranged on a gas delivery conduct or the like, branched to the downstream conduct element 30.

In some embodiments, the manual resuscitation bag 1 can comprise a handle (not shown) or the like for manually transporting it, for instance when used in a mobile emergency vehicle, such as an ambulance, a firefighting vehicle or the like.

As shown in Fig. 1, the downstream conduct elements 30 further includes an exhaust valve 35 with an exhaust port 36 for venting to the atmosphere, the CO₂-enriched gases expired by the patient and/or coming out of the lungs of the patient. It is further provided mobile port-closing means 37, actuatable by the rescuer, cooperating with the exhaust port 36 of the exhaust valve 35 for at least partially closing said exhaust port 36 thereby controlling the flow of gas passing through the exhaust port 36 of the exhaust valve 35, especially CO₂-enriched gas coming out of the patient lungs.

Said mobile port-closing means 37 are arranged on or comprise a mobile support-structure actuatable by the user. Said mobile port-closing means 37 are configured for at least partially closing the exhaust port 36 of the exhaust valve 35 in response to an actuation of the mobile support-structure by the user, typically to a rotation, pivoting or translation of the support-structure.

In the embodiment shown in Figure 1, the mobile adjusting member 37 is rotatable, i.e. can be turned clockwise or counter-clockwise by a rescuer, and is further coupled to or arranged on the downstream conduct element 30. Nevertheless, other embodiments are possible, such as a pivoting or translating element, for instance a rigid curtain or the like, directly arranged on the exhaust valve 35.

More generally speaking, said mobile port-closing means 37 cooperate with the exhaust port 36 of the exhaust valve 35 for partially closing said exhaust port 36 thereby limiting the flow of gas passing through said exhaust port 36. Of course, in opposite, if no flow limitation is desired, the port-closing means 37 can be omitted or removed from the exhaust port 36 so that said exhaust port 36 is free, i.e. widely open, thereby letting a maximum gas exiting through said exhaust port 36, i.e. be vented to the atmosphere.

For instance, the mobile port-closing means 37 can comprise a closing flap or wall or the like that can partially occlude the exhaust port 36, when desired, for controlling the gas flow passage, i.e. section area, or the like and hence controlling the pressure prevailing in the downstream conduct element 30, such as a expiratory pressure.

The closing flap or wall carried by the support-structure that can be actuated by the rescuer, typically rotated/turned (i.e. pivot), between several angular positions comprising a first angular position (i.e. "fully open" position) wherein it does not occlude/close the exhaust port 36 of the exhaust valve 35 so that a maximum flow of gas can be released to the atmosphere through said exhaust port 36, for instance when the patient is ventilated with the bag 1, and a second angular position (i.e. "closed" position) wherein it does occlude/close almost all of the exhaust port 36 of the exhaust valve 35, for instance about 90 or 95% of it (i.e. of its surface area), so that a minimum flow of gas can be released to the atmosphere through said exhaust port 36, while CO₂-containing gas exists the lungs of the patient, thereby increasing the flow resistance for the patient. Of course other intermediary angular positions may also exist between said first angular position and second angular position, for more or less occluding/closing the exhaust port 36 of the exhaust valve 35.

In some embodiments, the closing flap of the mobile port-closing means 37 can be fixed to or carried by the outer wall of the rotatable support-structure. For instance, said closing flap or wall can be made in one-piece, for instance molded in one-piece, with the support-structure. According to other embodiments, the mobile port-closing means 37 can be fixed to another part of the manual resuscitation bag 1, in particular of the downstream conduct 30, i.e. not associated to or integral with the rotatable adjusting member 37.

Optionally, a flow-restriction element, such as a disk element carrying a calibrated orifice, can also be arranged into the downstream conduct element 30 for regulating the flow of gas provided by the flexible reservoir 10.

Of course, the manual resuscitation bag 1 according to the invention can also comprise additional elements or features.

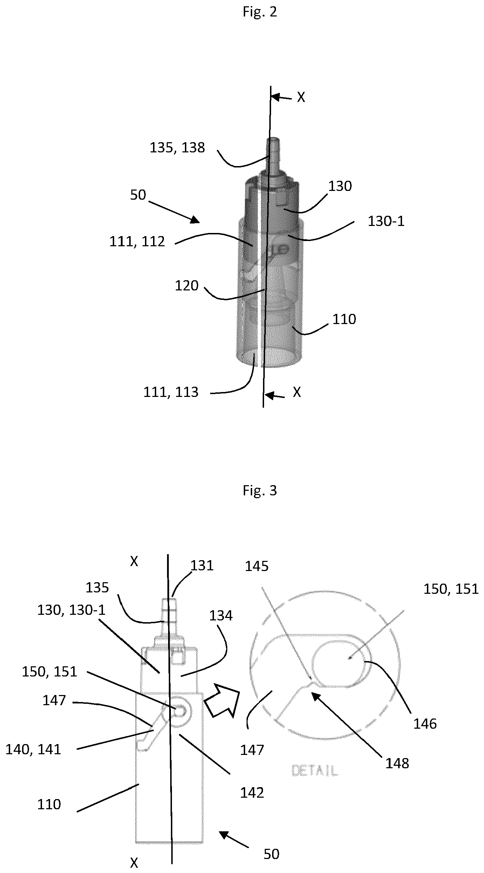

Figures 2 to 8 are different views of an embodiment of a venturi device 50 equipping a manual artificial respiration bag 1 according to the invention.

The venturi device 50 comprises an inner venturi nozzle 120 that is in fluid communication with the upstream conduct element 20, via the gas entry port 21 arranged in said upstream conduct element 20, for providing the desired gas to the upstream conduct element 20, namely oxygen or a mixture of oxygen and air, as explained below.

Figure 2 is a general view of a preferred embodiment of a venturi device 50 forming part of a manual artificial respiration bag 1 according to the present invention, showing in transparency the two main parts or sub-units of the venturi device 50 cooperating together, namely a main body 110 and a rear member 130, i.e. a gas-providing member, unit or part, which are mobile with respect to the other for controlling, i.e. selecting, the type of gas, i.e. desired gas, that has to be provided by the venturi device 50 to the deformable reservoir 10, namely either oxygen or an air/oxygen mixture.

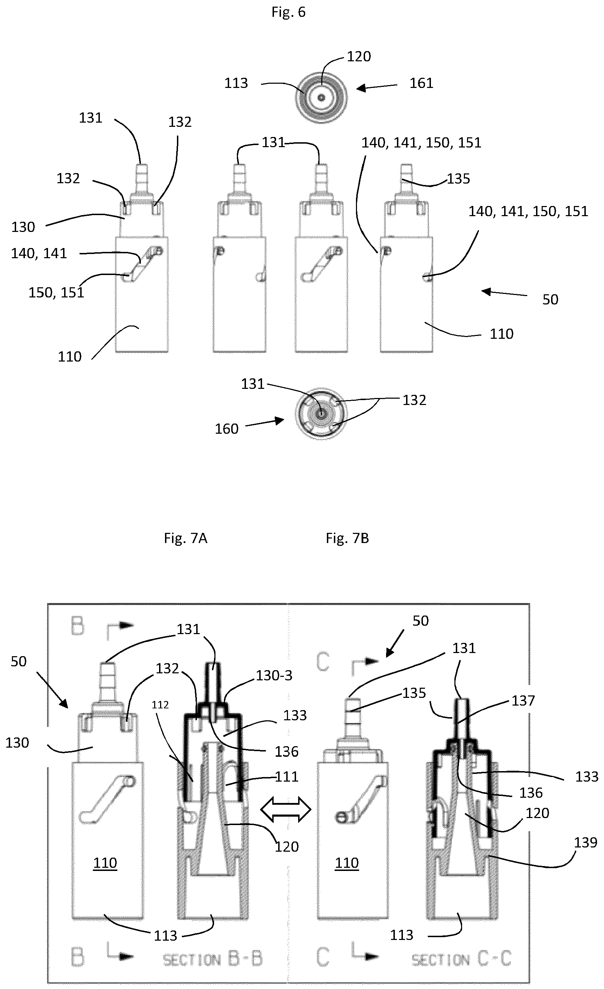

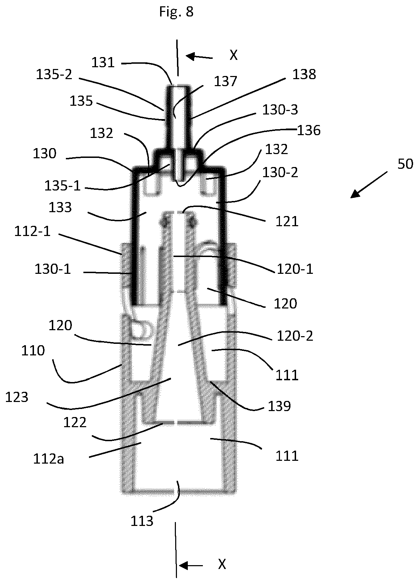

As shown in Fig. 7A&B and 8, the main body 110 comprises an inner compartment 111 comprising a venturi nozzle 120 co-axially arranged in said inner compartment 111. The inner compartment 111 is divided in 2 sub-compartments by an inner wall 139, namely an inlet chamber 112 or upstream chamber, and an outlet chamber 113 or downstream chamber. As the inner compartment 111 has a circular section, i.e. a cylindrical inner-shape, the inner wall 139 has a ring-shape.

The venturi nozzle 120 is arranged through the inner wall 139 that divides the inner compartment 111. It comprises a nozzle inlet 121 located on the side of the inlet chamber 112 and a nozzle outlet 122 located on the side of the outlet chamber 113, preferably in said outlet chamber 113. An inner nozzle channel 120-1, 120-2 is arranged between the nozzle inlet 121 and the nozzle outlet 122 for conveying the gas from the nozzle inlet 121 to the nozzle outlet 122.

More precisely, the venturi nozzle 120 comprises two successive channel portions forming the inner nozzle channel 120-1, 120-2 comprising a first channel portion 120-1 comprising the nozzle inlet 121 and having an inner cylindrical shape, and a second channel portion comprising the nozzle outlet 122 and having an inner divergent shape. The first channel portion 120-1 fluidly connects the nozzle inlet 121 to the second channel portion 120-2. According to another embodiment (not shown), the first channel portion 120-1 can have an inner convergent shape.

The second channel portion 120-2 is arranged between the first channel portion 120-1 and the nozzle outlet 122. As it comprises an inner divergent shape or profile, its inner diameter gradually increases toward the nozzle outlet 122.

The gas (i.e. O₂ or air/O₂) enters into the inner nozzle channel 120-1, 120-2 of the venturi nozzle 120 by the nozzle inlet 121, travels successively in the first 120-1 and then second 120-2 channel portions of the inner nozzle channel 120-1, 120-2, and flows out, i.e. leaves, of the venturi nozzle 120 by the nozzle outlet 122, before entering into the upstream conduct element 20, through the gas entry port 21.

The main body 110 of the venturi device 50 has an axially-elongated shape, i.e. along axis (XX), as shown in Fig. 7 (sectional view), in particular a tubular outer shape, e.g. cylindrical outer shape, but other outer shapes are possible. For instance, the outer diameter of the main body 110 is of about between 25 and 40 mm, and its length is of about between 60 and 110 mm.

Further, the inlet chamber 112 of the main body 110 is in fluid communication with the mixing chamber 133 of the rear member 130 that constitutes the second main part or sub-unit of the venturi device 50.

In the embodiment of Figure 7, the rear member 130 has a generally cup-like shape, but other shapes are possible. It comprises an (or several) oxygen inlet 131 for providing oxygen and one or preferably several air inlets 132 for providing air, and the mixing chamber 133 for mixing therein air with oxygen provided by said oxygen inlet 131. The oxygen inlet(s) 131 and the air inlets 132, for instance four air inlets 132, arranged around the oxygen inlet 131 are better visible in the top view 160 of Fig. 6.

In other words, the venturi device 50 comprises the main body 110 comprising the venturi nozzle 120 arranged therein, the oxygen inlet 131 for providing oxygen, the air inlets 132 for providing air, and the mixing chamber 133 for mixing the flow of oxygen provided by the oxygen inlet 131 and the flow of ambient air provided by air inlets 132, so as to provide a desired gas, namely only oxygen or an air/oxygen mixture, when such a mixture is required.

In this aime, the venturi device 50 further comprises gas selection means for selecting oxygen provided by the oxygen inlet 131 or an air/oxygen mixture provided by the mixing chamber 133 as a desired gas to be provided to the venturi nozzle 120, and subsequently to the upstream conduct element 20, through the gas entry port 21, of the manual artificial respiration bag 1 according to the present invention.

The gas selection means comprise the rear member 130 that is mobile with respect to the main body 110, upon actuation of a user, between (at least) a closed position (as shown in Fig. 7B), wherein the rear member 130 cooperates directly with the venturi nozzle 120 for providing oxygen to the venturi nozzle 120, in particular to the inner nozzle channel 120-1, 120-2 of the venturi nozzle 120, and an open position (as shown in Fig. 7A), wherein the rear member 130 cooperates with the venturi nozzle 120 for providing the air/oxygen mixture obtained in the mixing chamber 133 to the inner nozzle channel 120-1, 120-2 of the venturi nozzle 120.

In other words, in the closed position (Fig. 7B), the inner nozzle channel 120-1, 120-2 of the venturi nozzle 120 is fed with oxygen only, i.e. "pure" oxygen, whereas in the closed position (Fig. 7A), the inner nozzle channel 120-1, 120-2 of the venturi nozzle 120 is fed with a mixture of air and oxygen, i.e. an air/oxygen mixture, coming from the mixing chamber 133 of the rear member 130.

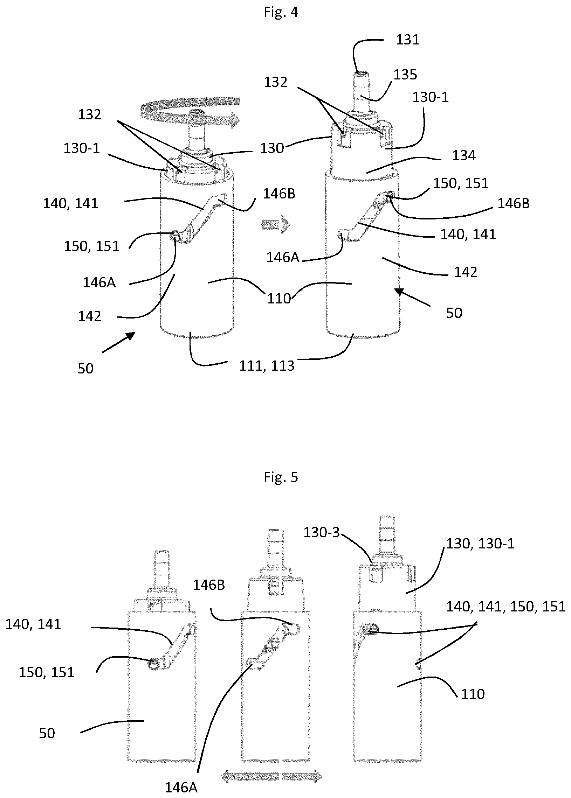

For avoiding or limiting accidental motions or actuations of the mobile parts, i.e. the rear member 130 that is mobile with respect to the main body 110, leading to wrong position settings and to a wrong-gas delivery, for instance oxygen in lieu of an air/O₂ mixture, or vice versa, the rear member 130 is preferably not mobile in translation, but rather according to a helicoidal motion with respect to the main body 110, as shown in Figures 4 & 5, for allowing the user, i.e. rescuer, medical staff member or the like, to manually select and set a desired open or closed position.

"According to a helicoidal motion" means that the selection or setting of the closed or open position is made by the user when turning, clockwise or counterclockwise (cf. Fig. 4 & 5), the rear member 130 with respect to the main body 110 of the venturi device 50, or vice versa, thereby obtaining an helicoidal motion of those parts 110, 130, one with respect to the other. In other words, it screws in and out. In other words, the rear member 130 is helicoidally mobile with respect to the main body 110 when manually-turned clockwise or counterclockwise by a user.

In other embodiments, the rear member 130 can be also mobile only in translation with respect to the main body 110 for selecting the closed or the open position. Nevertheless, a helicoidal motion is preferred as it avoids wrong or accidental motions of the mobile parts.

To achieve such a clockwise or counterclockwise motion of those parts, specific structures are provided on the rear member 130 and the main body 110 of the venturi device 50 equipping the manual artificial respiration bag 1 of the present invention.

In this aim, the main body 110 of the venturi device 50 comprises one or several elongated grooves 140 and the rear member 130 comprises one or several guiding members 50, or vice versa, preferably two elongated grooves 140 cooperating with two guiding members 150, as shown in Fig. 2-7A&B, each guiding member 150 being inserted into one of said elongated grooves 140, thereby cooperating with said elongated grooves 140 for guiding the helicoidal motion of the rear member 130 with respect to the main body 110, while the rear member 130 is actuated by a user, i.e. manually rotated/turned around axis (XX), with respect to the main body 110, or vice versa.

As shown in Fig. 5 & 6, the two elongated grooves 140 are arranged in the peripheral wall 142 of the main body 110 and are diametrically-opposed, whereas the two guiding members 150 are diametrically-oppositely arranged on the peripheral surface of the rear member 130. The elongated grooves 140 constitute guides or paths that guide the guiding members 150, when the venturi device 50 is manually-actuated by the user, i.e. turned clockwise or counterclockwise as shown in Fig. 4 & 5.

The elongated grooves 140 are preferably elongated slots 141 traversing the peripheral wall 142 of the main body 110, in particular the inlet chamber 112 of the main body 110, i.e. traversing slots 141 or the like.

Further, the guiding members 150 comprise or are protrusions 151 arranged on the peripheral outer surface 134 of the rear member 130, preferably the peripheral outer surface 134 of the front portion 130-1 of the rear member 130. Each protrusion 151 forms a dent, a pin, a finger or any other similar small structure projecting away from the peripheral outer surface 134 of the rear member 130.

As shown in Fig. 4-6, the elongated grooves 140 of the main body 110, in particular the slots 141, comprise a curved linear portion 147, i.e. a linear segment, arranged in the cylindrical peripheral wall 142 of the main body 110, which is terminated by two end lodgings 146A, 146B that correspond to the open (146B) and closed (146A) positions of the venturi device 50.

The end lodgings 146A, 146B are sized and configured for receiving or lodging the guiding members 150, in particular the protrusions 151. In other words, the protrusions 151 cooperate with the end lodgings 146A, 146B for setting the venturi device 50 in the open (146B) or closed (146A) positions as illustrated in Figure 3, thereby providing an oxygen/air mixture or only oxygen to the manual artificial respiration bag 1 of the present invention, via the inner venturi nozzle 120 and through the gas entry port 21 of the upstream conduct element 20, which are in fluid communication.

The curved linear portion 147 of each elongated groove 140, preferably of each elongated slot 141, is diagonally-arranged in the peripheral wall 142 of the main body 110, namely in the peripheral wall of the inlet chamber 112 of the main body 110 as shown in Fig. 4-6.

For ensuring a stable maintaining of the venturi device 50 in its open or closed positions, it is provided, in each end lodging 146A, 146B, an abutment 145 as visible in Figure 3, that is located at a site of junction 148 between of the curved linear portion 147 of the elongated grooves/slots 140, 141 and each end lodgings 146A, 146B.

As shown in Figure 3, said abutments 145 can be little wall structures or similar projecting in each end lodging 146A, 146B. The role of those abutments 145 is to block or to retain the protrusions 151 in the end lodgings 146A, 146B for setting the venturi device 50 in the open (146B) or in the closed (146A) positions as illustrated in Figures 3-6. In other words, those abutments 145 are safety blocks or the like.

For instance, when the venturi device 50 in the closed position with the two protrusions 151 lodged and blocked in the first end lodgings 146A corresponding to said closed position, as illustrated in Fig. 4 (left side), and has to be set in the open position, the user actuates, namely rotates counterclockwise, for instance the rear member 130 so that the two protrusions 151 are helicoidally guided by the two slots 141, in particular the linear curved portions of the slots 141, until they reach the second end lodgings 146B corresponding to the open position, as illustrated in Fig. 4 (right side).

Once lodged in the second end lodgings 146B, the two protrusions 151 are blocked and retained therein by the abutments 145, as better shown in Fig. 3, that shows an enlarged view of a second end lodging 146B with a protrusion 151 retained therein by an abutment 145. The abutments 45 prohibit that the protrusions 151 accidentally get out of the end lodgings 146A, 146B thereby ensuring stable open or closed positions of the venturi device 50.

For instance, the elongated grooves 140, e.g. the elongated slots 141, each have a length of about between 20 and 30 mm, and/or a width of about between 3.5 and 5 mm. Further, the guiding members 150, e.g. the protrusions 151, have a height of about between 1.5 and 3 mm.

Furthermore, as visible in Fig. 7A&7B and 8, for allowing the rear member 130 to rotate with respect to the main body 110 of the venturi device 50, a front portion 130-1 of the rear member 130 is inserted into at least a part of the inlet chamber 112 of the main body 110.

Further, said front portion 130-1 of the rear member 130 is externally cylindrical, i.e. tubular, and at least a part 112-1 of the inlet chamber 112 of the main body 110 is internally cylindrical, so that said cylindrical front portion 130-1 of the rear member 130 can be rotate in said cylindrical part 112-1 of the inlet chamber 112 of the main body 110. Preferably, the outer diameter Do of said front portion 130-1 of the rear member 130 is less but approximately equal to the inner diameter Di of the cylindrical part 112-1 of the inlet chamber 112 of the main body 110 so that the helicoidal motion is allowed, i.e. Do<Di, according to the embodiment shown in Figures 2-8.

Generally speaking, the front portion 130-1 of the rear member 130 delimits at least a part of the mixing chamber 133 used for mixing the oxygen flow and the air flow(s), when required. More precisely, the rear member 130 comprises a hollow tubular portion 130-2 closed at one end by a blind bottom 130-3, i.e. it has a cup-like shape or similar as better shown in Fig. 8. The other end of the tubular portion 130-2 is open toward the inlet chamber 112 of the main body 110. For instance, the rear member 130 has an outer diameter of about between 20 and 40 mm, and a length of about between 40 and 70 mm.

Further, the rear member 130 comprises, i.e. is traversed by, an oxygen feeding conduct 135 comprising an outer conduct portion 135-2, i.e. upstream portion, and an inner conduct portion 135-1, i.e. downstream portion, for providing oxygen gas to the venturi device 50.

The oxygen feeding conduct 135 is axially-arranged (XX axis) through the blind bottom 130-3 of the rear member 130, i.e. it traverses the blind bottom 130-3. The outer conduct portion 135-2 of the oxygen feeding conduct 135 comprises the oxygen inlet 131, at its distal free end, and is located outside of the venturi device 50.

Further, the inner conduct portion 135-1 of the oxygen feeding conduct 135 comprises an oxygen delivery port 136 for providing oxygen located at its free end.

The oxygen feeding conduct 135 is axially-traversed by a lumen 137 for conveying oxygen gas from the oxygen inlet 131 to the oxygen delivery port 136 thereby providing oxygen coming from an external oxygen source, such as a gas cylinder, a gas line or any other O₂ source.

More precisely, the outer conduct portion 135-2 of the oxygen feeding conduct 135 also comprises connector means 138, such as a specific outer design, for fluidly connecting the oxygen line 60 thereto, as shown in Fig. 1, that is fed by an external oxygen source (not shown), such as an oxygen gas cylinder or an oxygen wall-plug fed by a gas network.

As shown in Fig. 7A&7B and 8, the inner conduct portion 135-1 of the oxygen feeding conduct 135 is arranged so as to axially-project into the mixing chamber 133, whereas the outer conduct portion 135-2 of the oxygen feeding conduct 135 is arranged so as to axially-project outwardly of the rear member, i.e. outside of it.

The oxygen feeding conduct 135 is in fluid communication, in particular via the lumen 137 of the inner and outer conduct portions 135-1, 135-2, with the mixing chamber 133, when the rear member 130 is in the open position as shown in Fig. 7A and 8, for mixing air and oxygen, and providing such an O₂/air mixture to the venturi nozzle 120.

In contrast, the oxygen feeding conduct 135 is in fluid communication, in particular via the lumen 137, with the inner nozzle channel 120-1, 120-2 of the venturi nozzle 120, when the rear member 130 is in the closed position as shown in Fig. 7B. In the closed position, the proximal free end of the inner conduct portion 135-1 of the oxygen feeding conduct 135 penetrates in the inner nozzle channel 120-1, 120-2 of the venturi nozzle 120, through the nozzle inlet 121, to deliver oxygen directly into the venturi nozzle 120, subsequently to the upstream conduct element 20, via the gas entry port 21, of the manual artificial respiration bag 1 according to the invention, as shown in Fig. 1.

Thanks to the improved manual artificial respiration bag 1 of the present invention, it is possible to operate a more accurate control of the proportion of oxygen provided to the patient, especially when the proportion of oxygen has to be less than 100 vol.%, typically less than 80 vol.%, for instance of about 50 vol.%, especially in case of ROSC while ventilating a person having had a cardiac arrest.

Indeed, the venturi device integrated in the manual respiration bag 1 of the invention can provide at least two accurate and precise levels of FiO₂ for ventilating persons suffering from a cardiac arrest or from another respiratory pathology or medical condition requiring at least two different amounts of oxygen, i.e. various FiO₂, in particular a first amount of 100 vol% of oxygen, i.e. pure oxygen flow, and a second (or more) amount of oxygen that is less than 100 vol.%, resulting from the dilution of oxygen with ambient air in the mixing chamber 133 of the venturi device, for instance a quantity of oxygen of about 50 vol%.

The venturi system is designed for delivering a fixed amount of oxygen in the air/oxygen mixture obtained in the mixing chamber 133. In this goal, the dimensions of the air inlet 132, the nozzle inlet 121 and nozzle outlet 136 for instance can be chosen and set to get the desired FiO₂ level, for instance a FiO₂ level of 50 vol.%. The dilution rate is controlled by controlling or adjusting the amount of air passing through the mixing chamber 133 by venturi effect, which depends on the O₂ flow. Thus, with an O₂ flow of at least 5L/min, the FiO₂ will remain stable, for instance a FiO₂ of 50 vol.%.

Generally speaking, a manual artificial respiration bag 1 according to the present invention can be used in various situations, for instance for resuscitating a person in state of cardiac arrest or the like, or for ventilation a person during transportation from one place to another place, in the field, in hospital, at the patient's home, in emergency vehicles or in any other place.

## Claims

1. Manual artificial respiration bag (1) comprising:
- a deformable bag (10) comprising a gas inlet (11), a gas outlet (12) and an inner volume (13) for a respiratory gas,
- an upstream conduct element (20) fluidly connected to the gas inlet (11) of the deformable bag (10), and comprising a gas entry port (21), and
- a downstream conduct element (30) fluidly connected to the gas outlet (12) of the deformable bag (10), comprising an exhaust valve (35) comprising an exhaust port (36),
- a venturi device (50) comprising :
▪ a main body (110) comprising a venturi nozzle (120),
▪ at least one oxygen inlet (131) for providing oxygen,
▪ at least one air inlet (132) for providing air, and
▪ a mixing chamber (133) for mixing therein oxygen provided by said oxygen inlet (131) and air provided by said at least one air inlet (132), thereby obtaining an air/oxygen mixture,
and wherein the venturi nozzle (120) is in fluid communication with the upstream conduct element (20), via the gas entry port (21) arranged in said upstream conduct element (20), for providing the desired gas to the upstream conduct element (20),
**characterized in that**:
- the venturi device (50) further comprises gas selection means (110, 130) for selecting oxygen provided by said at least one oxygen inlet (131) or an air/oxygen mixture provided by said mixing chamber (133) as a desired gas to be provided to the venturi nozzle (120),
- the gas selection means (110, 130) of the venturi device (50) comprise a rear member (130) comprising said at least one oxygen inlet (131), said at least one air inlet (132), and said mixing chamber (133), said rear member (130) being mobile with respect to the main body (110) between at least :
▪ a closed position wherein the rear member (130) provides oxygen as the desired gas to the venturi nozzle (120), and
▪ an open position wherein the rear member (130) provides an air/oxygen mixture as the desired gas to the venturi nozzle (120),
- the rear member (130) of the gas selection means (110, 130) is mobile according to a helicoidal motion with respect to the main body (110) of the venturi device (50) for allowing a user to manually select the desired open or the closed position,
- the main body (110) of the venturi device (50) comprises at least one elongated groove (140) and the rear member (130) comprises at least one guiding member (150) comprising at least one protrusion (151), or vice versa, said at least one guiding member (150) being inserted into said at least one elongated groove (140) so as to cooperate with said at least one elongated groove (140) for guiding the helicoidal motion of the rear member (130) with respect to the main body (110), while actuated by a user,
- each elongated groove (140) of the main body (110) comprises a curved linear portion (147) terminated by two end lodgings (146A, 146B),
- each end lodging (146A, 146B) comprises an abutment (145), the end lodgings (146A, 146B) and the abutments (145) cooperating with said at least one protrusion (151) for setting the venturi device (50) in the open (146B) or in the closed (146A) positions, and
- the open (146B) and the closed (146A) positions are stable positions.

2. Manual artificial respiration bag according to Claim 1, **characterized in that** the main body (110) of the venturi device (50) comprises an inner compartment (111) containing the venturi nozzle (120).

3. Manual artificial respiration bag according to any one of the preceding claims, **characterized in that** the main body (110) of the venturi device (50) comprises at least two elongated grooves (140) and the rear member (130) comprises at least two guiding member (150), said at least two elongated grooves cooperating with at least two guiding members.

4. Manual artificial respiration bag according to any one of the preceding claims, **characterized in that** the rear member (130) comprises an oxygen feeding conduct (135).

5. Manual artificial respiration bag according to claim 4, **characterized in that** the oxygen feeding conduct (135) is axially-arranged in the rear member (130).

6. Manual artificial respiration bag according to claim 4, **characterized in that**, in the closed position, a proximal free end of an inner conduct portion (135-1) of the oxygen feeding conduct (135) of the rear member (130) is inserted into a first portion (120-1) of an inner nozzle channel (120-1, 120-2) of the venturi nozzle (120) so as to deliver oxygen directly into the venturi nozzle (120).

7. Manual artificial respiration bag according to claims 4 to 6, **characterized in that**, in the open position, the proximal free end of the inner conduct portion (135-1) of the oxygen feeding conduct (135) of the rear member (130) is positioned into the mixing chamber (133) for providing a flow of oxygen to the mixing chamber (133) so as to generate an air/oxygen mixture into said mixing chamber (133).

8. Manual artificial respiration bag according to claim 3, **characterized in that**
- the two elongated grooves (140) are diametrically-oppositely arranged on the main body (110) and
- the two guiding members (150) are diametrically-oppositely arranged on the rear member (130).

9. Manual artificial respiration bag according to claims 3 or 8, **characterized in that**
- the elongated grooves (140) are elongated traversing slots (141) traversing the peripheral wall (142) of the main body (110), and
- the guiding members (150) comprise or are protrusions (151) arranged on the peripheral outer surface (134) of the rear member (130).

10. Manual artificial respiration bag according to claim 1, **characterized in that** it further comprises an upstream one-way valve arranged into the upstream conduct element (20).

11. Manual artificial respiration bag according to claim 1, **characterized in that** the upstream conduct element (20) further comprises a PEP exhaust valve (22) fluidly communicating with the ambient atmosphere for venting gas to the atmosphere when the gas pressure, into the upstream conduct element (20), exceeds a given pressure threshold,

12. Manual artificial respiration bag according to claim 11, **characterized in that** the PEP exhaust valve (22) comprises PEP-setting means for setting the desired pressure threshold.

13. Manual artificial respiration bag according to claim 1, **characterized in that**:
- the upstream conduct element (20) further comprises a reservoir port (20-1) for fluidly connecting a flexible gas reservoir (40) thereto, and/or
- the downstream conduct element (30) further comprises an interface port (32) for fluidly connecting a respiratory interface thereto.

14. Manual artificial respiration bag according to claim 1, **characterized in that** it further comprises an upstream one-way valve arranged into the upstream conduct element (20) configured for allowing a flow of respiratory gas to pass through said upstream one-way valve only toward the deformable bag (2).

15. Manual artificial respiration bag according to any one of the preceding claims, **characterized in that** it further comprises :
- a flexible gas reservoir (40) fluidly connected to the reservoir port (20-1) of the upstream conduct element (20), and/or
- a respiratory interface fluidly connected the interface port (32) of the downstream conduct element (30), and/or
- an oxygen source fluidly connected to the venturi device (50).

## Patentansprüche

1. Manueller Beatmungsbeutel (1) mit:
- einen verformbaren Beutel (10) mit einem Gaseinlass (11), einem Gasauslass (12) und einem Innenvolumen (13) für ein Atemgas,
- ein stromaufwärts gelegenes Leitungselement (20), das fluidisch mit dem Gaseinlass (11) des verformbaren Beutels (10) verbunden ist und eine Gaseintrittsöffnung (21) umfasst, und
- ein stromabwärts gelegenes Leitungselement (30), das fluidisch mit dem Gasauslass (12) des verformbaren Beutels (10) verbunden ist und ein Auslassventil (35) mit einer Auslassöffnung (36) umfasst,
- eine Venturi-Vorrichtung (50), die umfasst:
▪ einen Hauptkörper (110) mit einer Venturi-Düse (120),
▪ mindestens einen Sauerstoffeinlass (131) zur Zufuhr von Sauerstoff,
▪ mindestens einen Lufteinlass (132) zur Zufuhr von Luft und
▪ eine Mischkammer (133) zum Mischen des von dem Sauerstoffeinlass (131) zugeführten Sauerstoffs und der von dem mindestens einen Lufteinlass (132) zugeführten Luft, wodurch ein Luft/Sauerstoff-Gemisch erhalten wird,
und wobei die Venturidüse (120) über die in dem stromaufwärtigen Leitungselement (20) angeordnete Gaseinlassöffnung (21) in Fluidverbindung mit dem stromaufwärtigen Leitungselement (20) steht, um das gewünschte Gas dem stromaufwärtigen Leitungselement (20) zuzuführen,
**dadurch gekennzeichnet, dass**:
- die Venturi-Vorrichtung (50) ferner Gasauswahlmittel (110, 130) umfasst, um den von dem mindestens einen Sauerstoffeinlass (131) bereitgestellten Sauerstoff oder ein von der Mischkammer (133) bereitgestelltes Luft/Sauerstoff-Gemisch als gewünschtes Gas auszuwählen, das der Venturi-Düse (120) zugeführt werden soll,
- die Gasauswahleinrichtung (110, 130) der Venturi-Vorrichtung (50) ein hinteres Element (130) umfasst, das den mindestens einen Sauerstoffeinlass (131), den mindestens einen Lufteinlass (132) und die Mischkammer (133) umfasst, wobei das hintere Element (130) in Bezug auf den Hauptkörper (110) zwischen mindestens folgenden Positionen beweglich ist:
▪ einer geschlossenen Position, in der das hintere Element (130) Sauerstoff als gewünschtes Gas zur Venturidüse (120) liefert, und
▪ einer offenen Position, in der das hintere Element (130) ein Luft/SauerstoffGemisch als gewünschtes Gas an die Venturidüse (120) liefert,
- das hintere Element (130) der Gasauswahleinrichtung (110, 130) in Bezug auf den Hauptkörper (110) der Venturi-Vorrichtung (50) in einer schraubenförmigen Bewegung beweglich ist, damit ein Benutzer manuell die gewünschte offene oder geschlossene Position auswählen kann,
- der Hauptkörper (110) der Venturi-Vorrichtung (50) mindestens eine längliche Nut (140) aufweist und das hintere Element (130) mindestens ein Führungselement (150) mit mindestens einem Vorsprung (151) aufweist, oder umgekehrt, wobei das mindestens eine Führungselement (150) in die mindestens eine längliche Nut (140) eingesetzt ist, um mit der mindestens einen länglichen Nut (140) zusammenzuwirken, um die schraubenförmige Bewegung des hinteren Elements (130) in Bezug auf den Hauptkörper (110) zu führen, während es von einem Benutzer betätigt wird,
- jede längliche Nut (140) des Hauptkörpers (110) einen gekrümmten linearen Abschnitt (147) umfasst, der durch zwei Endaufnahmen (146A, 146B) begrenzt ist,
- jede Endaufnahme (146A, 146B) umfasst ein Widerlager (145), wobei die Endaufnahmen (146A, 146B) und die Anschläge (145) mit dem mindestens einen Vorsprung (151) zusammenwirken, um die Venturi-Vorrichtung (50) in die offene (146B) oder in die geschlossene (146A) Position zu bringen, und
- die geöffnete (146B) und die geschlossene (146A) Position stabile Positionen sind.

2. Manueller Beatmungsbeutel gemäß Anspruch 1, **dadurch gekennzeichnet, dass** der Hauptkörper (110) der Venturi-Vorrichtung (50) ein Innenfach (111) umfasst, das die Venturi-Düse (120) enthält.

3. Manueller Beatmungsbeutel gemäß einem der vorstehenden Ansprüche, **dadurch gekennzeichnet, dass** der Hauptkörper (110) der Venturi-Vorrichtung (50) mindestens zwei längliche Nuten (140) umfasst und das hintere Element (130) mindestens zwei Führungselemente (150) umfasst, wobei die mindestens zwei länglichen Nuten mit den mindestens zwei Führungselementen zusammenwirken.

4. Manueller Beatmungsbeutel gemäß einem der vorstehenden Ansprüche, **dadurch gekennzeichnet, dass** das hintere Element (130) eine Sauerstoffzufuhrleitung (135) umfasst.

5. Manueller Beatmungsbeutel gemäß Anspruch 4, **dadurch gekennzeichnet, dass** die Sauerstoffzufuhrleitung (135) axial im hinteren Element (130) angeordnet ist.

6. Manueller Beatmungsbeutel gemäß Anspruch 4, **dadurch gekennzeichnet, dass** in der geschlossenen Position ein proximales freies Ende eines inneren Leitungsabschnitts (135-1) der Sauerstoffzufuhrleitung (135) des hinteren Elements (130) in einen ersten Abschnitt (120-1) eines inneren Düsenkanals (120-1, 120-2) der Venturi-Düse (120) eingeführt ist, um Sauerstoff direkt in die Venturi-Düse (120) zu leiten.

7. Manueller Beatmungsbeutel gemäß den Ansprüchen 4 bis 6, **dadurch gekennzeichnet, dass** in der geöffneten Position das proximale freie Ende des inneren Leitungsabschnitts (135-1) der Sauerstoffzufuhrleitung (135) des hinteren Elements (130) in der Mischkammer (133) positioniert ist, um einen Sauerstoffstrom zur Mischkammer (133) zu erzeugen, um ein Luft/Sauerstoff-Gemisch in der Mischkammer (133) zu erzeugen.

8. Manueller Beatmungsbeutel gemäß Anspruch 3, **dadurch gekennzeichnet, dass** :
- die beiden länglichen Nuten (140) diametral gegenüberliegend am Hauptkörper (110) angeordnet sind und
- die beiden Führungselemente (150) diametral gegenüberliegend am hinteren Element (130) angeordnet sind.

9. Manueller Beatmungsbeutel gemäß Anspruch 3 oder 8, **dadurch gekennzeichnet, dass** :
- die länglichen Nuten (140) längliche, die Umfangswand (142) des Hauptkörpers (110) durchquerende Schlitze (141) sind, und
- die Führungselemente (150) umfassen oder sind Vorsprünge (151), die an der peripheren Außenfläche (134) des hinteren Elements (130) angeordnet sind.

10. Manueller Beatmungsbeutel nach Anspruch 1, **dadurch gekennzeichnet, dass** er ferner ein stromaufwärts angeordnetes Einwegventil umfasst, das in das stromaufwärts angeordnete Leitungselement (20) angeordnet ist.

11. Manueller Beatmungsbeutel gemäß Anspruch 1, **dadurch gekennzeichnet, dass** das stromaufwärtige Leitungselement (20) ferner ein PEP-Auslassventil (22) umfasst, das in Fluidverbindung mit der Umgebungsatmosphäre steht, um Gas in die Atmosphäre abzulassen, wenn der Gasdruck im stromaufwärtigen Leitungselement (20) einen bestimmten Druckschwellenwert überschreitet.

12. Manueller Beatmungsbeutel gemäß Anspruch 11, **dadurch gekennzeichnet, dass** das PEP-Auslassventil (22) eine PEP-Einstellvorrichtung zum Einstellen des gewünschten Druckgrenzwerts umfasst.

13. Manueller Beatmungsbeutel gemäß Anspruch 1, **dadurch gekennzeichnet, dass**:
- das stromaufwärtige Leitungselement (20) ferner einen Reservoiranschluss (20-1) umfasst, um einen flexiblen Gasbehälter (40) fluidisch damit zu verbinden, und/oder
- das stromabwärts gelegene Leitungselement (30) ferner einen Schnittstellenanschluss (32) zum fluiden Verbinden einer Atemschnittstelle damit umfasst.

14. Manueller Beatmungsbeutel gemäß Anspruch 1, **dadurch gekennzeichnet, dass** er ferner ein stromaufwärts gelegenes Einwegventil umfasst, das in dem stromaufwärts gelegenen Leitungselement (20) angeordnet ist und so konfiguriert ist, dass es einen Fluss von Atemgas nur in Richtung des verformbaren Beutels (2) durch das stromaufwärts gelegene Einwegventil hindurchzulässt.

15. Manueller Beatmungsbeutel gemäß einem der vorstehenden Ansprüche, **dadurch gekennzeichnet, dass** er ferner umfasst:
- einen flexiblen Gasbehälter (40), der fluidisch mit dem Behälteranschluss (20-1) des stromaufwärts gelegenen Leitungselements (20) verbunden ist, und/oder
- eine Atemschnittstelle, die fluidisch mit dem Schnittstellenanschluss (32) des stromabwärts gelegenen Leitungselements (30) verbunden ist, und/oder
- eine mit der Venturi-Vorrichtung (50) fluidisch verbundene Sauerstoffquelle.

## Revendications

1. Ballon manuel de respiration artificielle (1) comprenant :
- un ballon déformable (10) comprenant une entrée de gaz (11), une sortie de gaz (12) et un volume interne (13) pour un gaz respiratoire,
- un élément de conduite amont (20) relié de manière fluide à l'entrée de gaz (11) du sac déformable (10) et comprenant un orifice d'entrée de gaz (21), et
- un élément de conduite aval (30) relié de manière fluidique à la sortie de gaz (12) du ballon déformable (10), comprenant une soupape d'échappement (35) comprenant un orifice d'échappement (36),
- un dispositif à venturi (50) comprenant :
▪ un corps principal (110) comprenant une buse venturi (120),
▪ au moins une entrée d'oxygène (131) pour fournir de l'oxygène,
▪ au moins une entrée d'air (132) pour fournir de l'air, et
▪ une chambre de mélange (133) pour y mélanger l'oxygène fourni par ladite entrée d'oxygène (131) et l'air fourni par ladite au moins une entrée d'air (132), obtenant ainsi un mélange air/oxygène,
et dans lequel la buse venturi (120) est en communication fluidique avec l'élément de conduite amont (20), via l'orifice d'entrée de gaz (21) disposé dans ledit élément de conduite amont (20), pour fournir le gaz souhaité à l'élément de conduite amont (20),
**caractérisé en ce que** :
- le dispositif à venturi (50) comprend en outre des moyens de sélection de gaz (110, 130) pour sélectionner l'oxygène fourni par ladite au moins une entrée d'oxygène (131) ou un mélange air/oxygène fourni par ladite chambre de mélange (133) comme gaz souhaité à fournir à la buse à venturi (120),
- les moyens de sélection de gaz (110, 130) du dispositif à venturi (50) comprennent un élément arrière (130) comprenant ladite au moins une entrée d'oxygène (131), ladite au moins une entrée d'air (132), et ladite chambre de mélange (133), ledit élément arrière (130) étant mobile par rapport au corps principal (110) entre au moins :
▪ une position fermée dans laquelle l'élément arrière (130) fournit de l'oxygène comme gaz souhaité à la buse venturi (120), et
▪ une position ouverte dans laquelle l'élément arrière (130) fournit un mélange air/oxygène comme gaz souhaité à la buse à venturi (120),
- l'élément arrière (130) du moyen de sélection de gaz (110, 130) est mobile selon un mouvement hélicoïdal par rapport au corps principal (110) du dispositif à venturi (50) pour permettre à un utilisateur de sélectionner manuellement la position ouverte ou fermée souhaitée,
- le corps principal (110) du dispositif à venturi (50) comprend au moins une rainure allongée (140) et l'élément arrière (130) comprend au moins un élément de guidage (150) comprenant au moins une saillie (151), ou vice versa, ledit au moins un élément de guidage (150) étant inséré dans ladite au moins une rainure allongée (140) de manière à coopérer avec ladite au moins une rainure allongée (140) pour guider le mouvement hélicoïdal de l'élément arrière (130) par rapport au corps principal (110), lorsqu'il est actionné par un utilisateur,
- chaque rainure allongée (140) du corps principal (110) comprend une partie linéaire courbe (147) terminée par deux logements d'extrémité (146A, 146B),
- chaque logement d'extrémité (146A, 146B) comprend une butée (145), les logements d'extrémité (146A, 146B) et les butées (145) coopérant avec ladite au moins une saillie (151) pour mettre le dispositif à venturi (50) dans les positions ouverte (146B) ou fermée (146A), et
- les positions ouverte (146B) et fermée (146A) sont des positions stables.

2. Ballon manuel de respiration artificielle selon la revendication 1, **caractérisé en ce que** le corps principal (110) du dispositif Venturi (50) comprend un compartiment interne (111) contenant la buse Venturi (120).

3. Ballon manuel de respiration artificielle selon l'une quelconque des revendications précédentes, **caractérisé en ce que** le corps principal (110) du dispositif à venturi (50) comprend au moins deux rainures allongées (140) et l'élément arrière (130) comprend au moins deux éléments de guidage (150), lesdites au moins deux rainures allongées coopérant avec lesdits au moins deux éléments de guidage.

4. Ballon manuel de respiration artificielle selon l'une quelconque des revendications précédentes, **caractérisé en ce que** l'élément arrière (130) comprend un conduit d'alimentation en oxygène (135).

5. Ballon manuel de respiration artificielle selon la revendication 4, **caractérisé en ce que** le conduit d'alimentation en oxygène (135) est disposé axialement dans l'élément arrière (130).

6. Ballon manuel de respiration artificielle selon la revendication 4, **caractérisé en ce que**, en position fermée, une extrémité libre proximale d'une partie de conduit interne (135-1) du conduit d'alimentation en oxygène (135) de l'élément arrière (130) est insérée dans une première partie (120-1) d'un canal de buse interne (120-1, 120-2) de la buse à venturi (120) de manière à délivrer de l'oxygène directement dans la buse à venturi (120).

7. Ballon manuel de respiration artificielle selon les revendications 4 à 6, **caractérisé en ce que**, dans la position ouverte, l'extrémité libre proximale de la partie conductrice interne (135-1) du conduit d'alimentation en oxygène (135) de l'élément arrière (130) est positionnée dans la chambre de mélange (133) pour fournir un flux d'oxygène à la chambre de mélange (133) de manière à générer un mélange air/oxygène dans ladite chambre de mélange (133).

8. Ballon manuel de respiration artificielle selon la revendication 3, **caractérisé en ce que** :
- les deux rainures allongées (140) sont disposées de manière diamétralement opposée sur le corps principal (110) et
- les deux éléments de guidage (150) sont disposés de manière diamétralement opposée sur l'élément arrière (130).

9. Ballon manuel de respiration artificielle selon les revendications 3 ou 8, **caractérisé en ce que** :
- les rainures allongées (140) sont des fentes transversales allongées (141) traversant la paroi périphérique (142) du corps principal (110), et
- les éléments de guidage (150) comprennent ou sont des saillies (151) disposées sur la surface périphérique extérieure (134) de l'élément arrière (130).

10. Ballon manuel de respiration artificielle selon la revendication 1, **caractérisé en ce qu'**il comprend en outre une valve unidirectionnelle amont disposée dans l'élément de conduite amont (20).

11. Ballon manuel de respiration artificielle selon la revendication 1, **caractérisé en ce que** l'élément de conduite amont (20) comprend en outre une soupape d'échappement PEP (22) communiquant de manière fluide avec l'atmosphère ambiante pour évacuer le gaz vers l'atmosphère lorsque la pression du gaz dans l'élément de conduite amont (20) dépasse un seuil de pression donné.

12. Ballon manuel de respiration artificielle selon la revendication 11, **caractérisé en ce que** la soupape d'échappement PEP (22) comprend des moyens de réglage PEP pour régler le seuil de pression souhaité.

13. Ballon manuel de respiration artificielle selon la revendication 1, **caractérisé en ce que** :
- l'élément de conduite amont (20) comprend en outre un orifice de réservoir (20-1) pour y raccorder de manière fluide un réservoir de gaz flexible (40), et/ou
- l'élément de conduite aval (30) comprend en outre un orifice d'interface (32) pour raccorder de manière fluide une interface respiratoire à celui-ci.

14. Ballon manuel de respiration artificielle selon la revendication 1, **caractérisé en ce qu'**il comprend en outre une valve unidirectionnelle amont disposée dans l'élément de conduite amont (20) et configurée pour permettre à un flux de gaz respiratoire de passer à travers ladite valve unidirectionnelle amont uniquement vers le ballon déformable (2).

15. Ballon manuel de respiration artificielle selon l'une quelconque des revendications précédentes, **caractérisé en ce qu'**il comprend en outre :
- un réservoir de gaz flexible (40) relié de manière fluide au port de réservoir (20-1) de l'élément de conduite amont (20), et/ou
- une interface respiratoire reliée de manière fluide à l'orifice d'interface (32) de l'élément de conduite aval (30), et/ou
- une source d'oxygène reliée de manière fluide au dispositif venturi (50).
